(19) Europäisches Patentamt
European Patent Office
Office européen des brevets

(11) EP 0 772 653 B1

(12) **EUROPÄISCHE PATENTSCHRIFT**

(45) Veröffentlichungstag und Bekanntmachung des Hinweises auf die Patenterteilung:
**10.06.1998 Patentblatt 1998/24**

(21) Anmeldenummer: **95934075.3**

(22) Anmeldetag: **19.09.1995**

(51) Int Cl.[6]: **C09B 62/08**, C09B 62/507, C07C 309/47

(86) Internationale Anmeldenummer:
**PCT/EP95/03687**

(87) Internationale Veröffentlichungsnummer:
**WO 96/10610 (11.04.1996 Gazette 1996/16)**

(54) **REAKTIVE AZOFARBSTOFFE MIT EINER KUPPLUNGSKOMPONENTE AUS DER AMINONAPHTHALINREIHE**

REACTIVE AZO DYES WITH A COUPLER OF THE AMINONAPHTHALENE SERIES

COLORANTS AZOIQUES REACTIFS AVEC UN COPULATEUR DE LA SERIE DES AMINONAPHTALENES

(84) Benannte Vertragsstaaten:
**BE CH DE FR GB IT LI**

(30) Priorität: **30.09.1994 DE 4434989**

(43) Veröffentlichungstag der Anmeldung:
**14.05.1997 Patentblatt 1997/20**

(73) Patentinhaber: **BASF AKTIENGESELLSCHAFT**
**67056 Ludwigshafen (DE)**

(72) Erfinder:
- **DORNHAGEN, Jürgen**
  **D-67117 Limburgerhof (DE)**
- **KILBURG, Heike**
  **D-67346 Speyer (DE)**
- **PATSCH, Manfred**
  **D-67157 Wachenheim (DE)**
- **LÖFFLER, Hermann**
  **D-67346 Speyer (DE)**
- **SCHAFFER, Ortwin**
  **D-67063 Ludwigshafen (DE)**

(56) Entgegenhaltungen:

| | |
|---|---|
| **EP-A- 0 369 385** | **CH-A- 567 084** |
| **DE-B- 1 220 060** | **FR-A- 2 221 496** |
| **FR-A- 2 242 377** | **FR-A- 2 248 301** |
| **FR-A- 2 424 304** | **FR-A- 2 429 243** |
| **GB-A- 2 053 261** | **US-A- 4 036 825** |

- **JOURNAL OF THE AMERICAN CHEMICAL SOCIETY, Bd. 96, Nr. 19, 18.September 1974 DC US, Seiten 6195-6196, E.M.KOSOWER ET AL. 'Fluorescence of 2-N-Arylamino-6-naphthalenesulfonates in Glycerol'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 772 653 B1

Printed by Jouve, 75001 PARIS (FR)

## Beschreibung

Die vorliegende Erfindung betrifft neue Reaktivfarbstoffe der Formel I

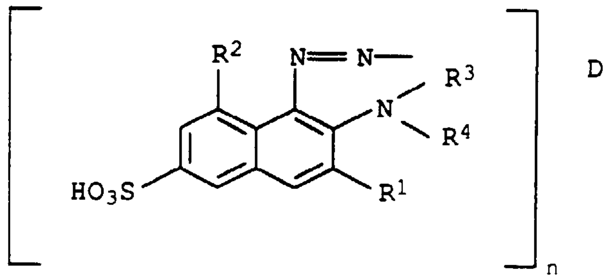 (I),

in der

n 1 oder 2,

$R^1$ Wasserstoff oder Hydroxysulfonyl,

$R^2$ Wasserstoff oder Hydroxy,

$R^3$ Carboxymethyl, Hydroxysulfonylmethyl, einen Rest der Formel $W^1$-$SO_2$-Y oder $W^2$(-$SO_2$-Y)$_2$, worin $W^1$ für $C_1$-$C_4$-Alkylen oder gegebenenfalls substituiertes Phenylen, $W^2$ für einen 3-wertigen Rest eines Benzolringes, der gegebenenfalls substituiert ist, und Y für Vinyl oder einen Rest der Formel $C_2H_4$-Q stehen, wobei Q die Bedeutung einer unter alkalischen Reaktionsbedingungen abspaltbaren Gruppe besitzt, Phenyl, das durch Carboxyl oder einen Rest der Formel CONH-$W^1$-$SO_2$-Y oder $SO_2$-Y, worin $W^1$ und Y jeweils die obengenannte Bedeutung besitzen, substituiert ist, oder wenn n 2 ist, auch Carboxy-$C_2$-$C_4$-alkyl oder Hydroxysulfonyl-$C_2$-$C_4$-alkyl,

$R^4$ Wasserstoff oder $R^3$ und $R^4$ zusammen mit dem sie verbindenden Stickstoffatom einen 5- oder 6-gliedrigen gesättigten heterocyclischen Rest, der weitere Heteroatome aufweisen kann, und

D , wenn n 1 ist, einen Rest der Formel

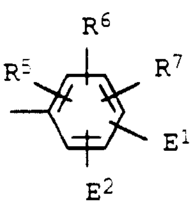

oder, wenn n 2 ist, einen Rest der Formel

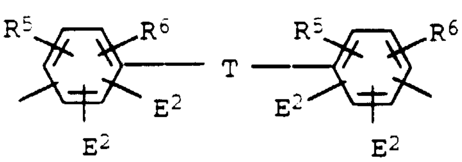 ,

worin $R^5$, $R^6$ und $R^7$ unabhängig voneinander jeweils für Wasserstoff, $C_1$-$C_4$-Alkyl, $C_1$-$C_4$-Alkoxy oder Hydroxysulfonyl, $E^1$ für einen heterocyclischen Ankerrest oder einen Ankerrest aus der aliphatischen Reihe, $E^2$ für Wasserstoff oder den Rest $E^1$ und T für ein Brückenglied stehen, bedeuten,

mit der Maßgabe, daß mindestens ein Ankerrest im Rest D vorhanden ist,

ihre Verwendung zum Färben oder Bedrucken von Hydroxygruppen oder Stickstoffatome aufweisenden organischen Substraten sowie neue Aminonaphthaline.

Aus der EP-A-369 385 sind Reaktivfarbstoffe auf Azobasis bekannt, die als Kupplungskomponente 1-Hydroxy-3-hydroxysulfonyl-7-(2-cyano-, 2-hydroxysulfonyl- oder 2-carbamoylethylamino)naphthalin aufweisen.

Weiterhin beschreiben die DE-A-2 154 942 und DE-A-2 163 389 Reaktivfarbstoffe dieser Klasse mit 1-Hydroxy-3-hydroxysulfonyl-7-methyl- oder phenylaminonaphthalin als Kupplungskomponente.

Aufgabe der vorliegenden Erfindung war es, neue Reaktivfarbstoffe, die sich von Phenylazonaphthalinfarbstoffen ableiten, bereitzustellen. Die neuen Farbstoffe sollten sich durch ein vorteilhaftes anwendungstechnisches Eigenschaftsprofil auszeichnen.

Demgemäß wurden die eingangs näher bezeichneten Reaktivfarbstoffe der Formel I gefunden.

Die neuen Reaktivfarbstoffe der Formel I sind jeweils in Form der freien Säure angegeben. Selbstverständlich werden auch deren Salze von den Patentansprüchen umfaßt.

Geeignete Kationen leiten sich von Metall- oder Ammoniumionen ab. Metallionen sind insbesondere die Lithium-, Natrium- oder Kaliumionen. Unter Ammoniumionen im erfindungsgemäßen Sinne sind unsubstituierte oder substituierte Ammoniumkationen zu verstehen. Substituierte Ammoniumkationen sind z.B. Monoalkyl-, Dialkyl-, Trialkyl-, Tetraalkyl- oder Benzyltrialkylammoniumkationen oder solche Kationen, die sich von stickstoffhaltigen 5- oder 6-gliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen geradkettiges oder verzweigtes $C_1$-$C_{20}$-Alkyl zu verstehen, das durch Hydroxylgruppen substituiert und/oder durch 1 bis 4 Sauerstoffatome in Etherfunktion unterbrochen sein kann.

Alle in der obengenannten Formel I auftretenden Alkyl- und Alkylenreste können sowohl geradkettig als auch verzweigt sein.

Wenn in der obengenannten Formel I substituierte Alkylgruppen auftreten, so weisen sie in der Regel 1 oder 2 Substituenten auf.

Wenn in der obengenannten Formel substituierte Phenyl-, Phenylenoder Benzoltriylgruppen auftreten, so können, sofern nicht anders vermerkt, z.B. $C_1$-$C_4$-Alkyl, $C_1$-$C_4$-Alkoxy, Halogen, Hydroxysulfonyl, Sulfamoyl oder $C_1$-$C_4$-Mono- oder Dialkylsulfamoyl als Substituenten in Betracht kommen. Sie weisen dann in der Regel 1 bis 3, vorzugsweise 1 oder 2 Substituenten auf.

Wenn die Reste $R^3$ und $R^4$ zusammen mit dem sie verbindenden Stickstoffatom einen 5- oder 6-gliedrigen gesättigten heterocyclischen Rest, der weitere Heteroatome aufweisen kann, bedeuten, so können dafür z.B. Pyrrolidinyl, Piperidinyl, Morpholinyl, Thiomorpholinyl, Thiomorpholinyl-S,S-dioxid, Piperazinyl oder N-($C_1$-$C_4$-Alkyl)piperazinyl, wie N-Methyl- oder N-Ethylpiperazinyl, in Betracht kommen.

Reste $R^5$, $R^6$ und $R^7$ sind z.B. Methyl, Ethyl, Propyl, Isopropyl, Butyl, Isobutyl, sec-Butyl, tert-Butyl, Methoxy, Ethoxy, Propoxy, Isopropoxy, Butoxy, Isobutoxy oder sec-Butoxy.

Reste $W^1$ sind z.B. $CH_2$, $(CH_2)_2$, $CH_2)_3$, $(CH_2)_4$, $CH(CH_3)CH_2$, $CH(CH_3)CH(CH_3)$ oder 1,2-, 1,3- oder 1,4-Phenylen.

Reste $W^2$ sind z.B. Benzol-1,2,3-, -1,3,4- oder -1,3,5-triyl.

Reste $R^3$ sind z.B. Hydroxysulfonylmethyl, 2-Hydroxysulfonylethyl, Carboxymethyl, 1- oder 2-Carboxyethyl, ein Rest der Formel $C_2H_4$-$SO_2$-Y oder $C_3H_6$-$SO_2$-Y, 2-, 3- oder 4-Carboxyphenyl oder ein Rest der Formel

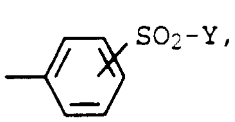 ,

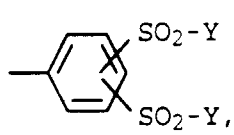 ,

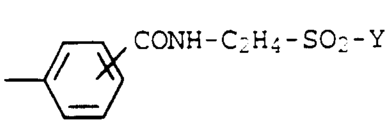

oder

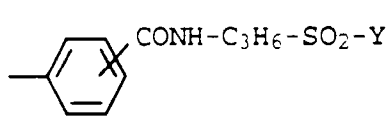 .

Der Rest Q steht für eine unter alkalischen Reaktionsbedingungen abspaltbare Gruppe. Solche Gruppen sind z. B. Chlor, Brom, $C_1$-$C_4$-Alkylsulfonyl, Phenylsulfonyl, $OSO_3H$, $SSO_3H$, $OP(O)(OH)_2$, $C_1$-$C_4$-Alkylsulfonyloxy, gegebenenfalls substituiertes Phenylsulfonyloxy, $C_1$-$C_4$-Alkanoyloxy, $C_1$-$C_4$-Dialkylamino oder ein Rest der Formel

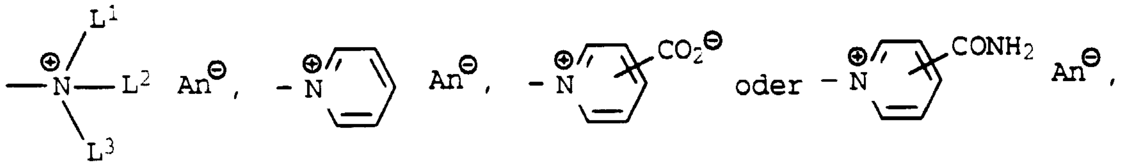

oder

wobei $L^1$, $L^2$ und $L^3$ unabhängig voneinander jeweils die Bedeutung von $C_1$-$C_4$-Alkyl oder Benzyl und $An^{\ominus}$ jeweils die Bedeutung eines Äquivalents eines Anions besitzen. Als Anionen können dabei z.B. Fluorid, Chlorid, Bromid, Iodid, Mono-, Di- oder Trichloracetat, Methansulfonat, Benzolsulfonat oder 2- oder 4-Methylbenzolsulfonat in Betracht kommen.

Ankerreste $E^1$ und $E^2$ sind solche, die mit den Hydroxygruppen oder Stickstoffatome enthaltenden Gruppen der zu behandelnden Substrate substitutiv oder additiv reagieren.

Daß der Ankerrest mit den betreffenden Gruppen in den Substraten, z.B. mit den Hydroxygruppen der Cellulose, substitutiv reagiert, bedeutet, daß die Austrittsgruppen oder -atome (z.B. Fluor oder Chlor) im Ankerrest durch die Hydroxygruppen der Cellulose gemäß folgendem Schema substituiert werden:

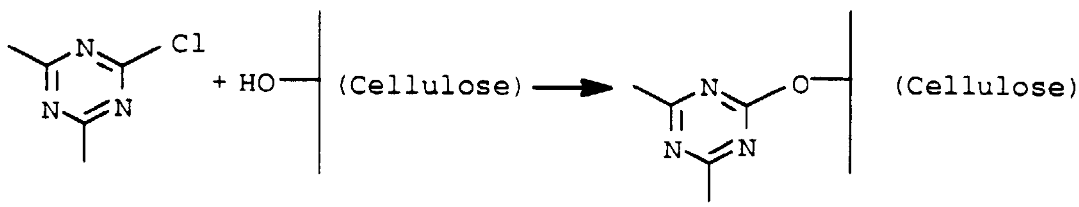

Daß der Ankerrest mit betreffenden Gruppen in den Substraten, z.B. mit Hydroxygruppen der Cellulose, additiv reagiert, bedeutet, daß die Hydroxygruppen der Cellulose gemäß folgendem Schema an den Ankerrest addiert werden:

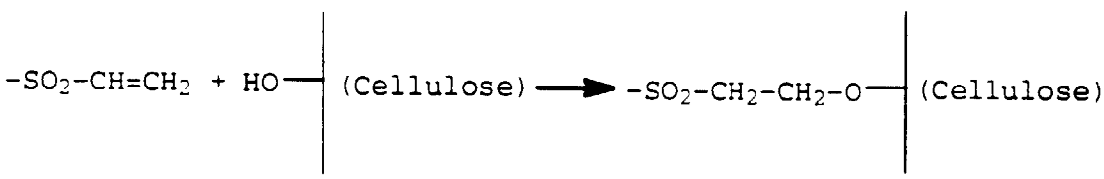

Heterocyclische Ankerreste $E^1$ und $E^2$ sind z.B. halogensubstituierte Reste von 1,3,5-Triazin, Chinoxalin, Phthalazin, Pyrimidin oder Pyridazon, oder der 2-Alkylsulfonylbenzthiazolrest.

Beispielhaft seien folgende heterocyclische Reste genannt:

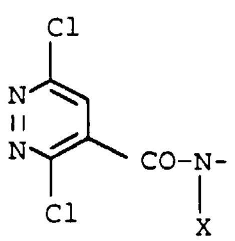 , 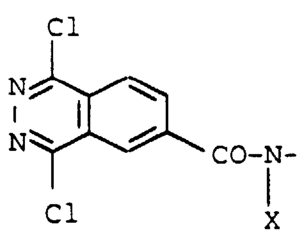 , 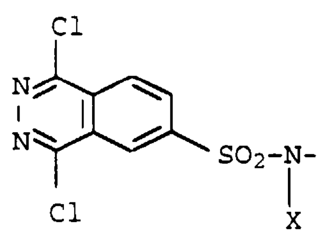 ,

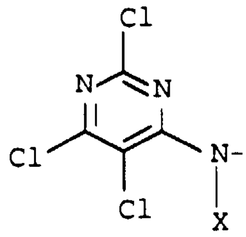

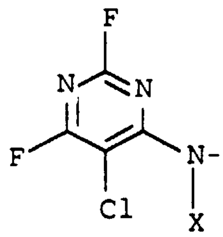 ,

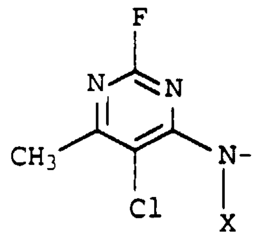 ,

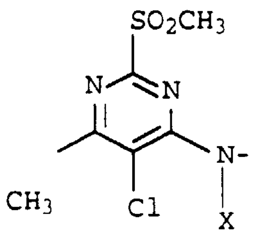

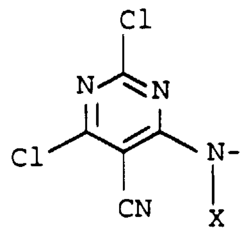 ,

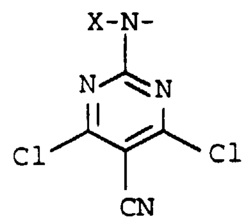 ,

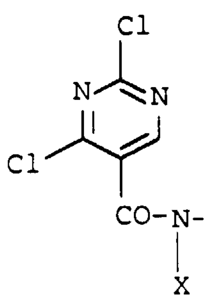

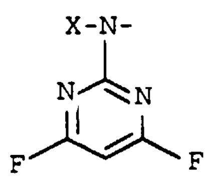 ,

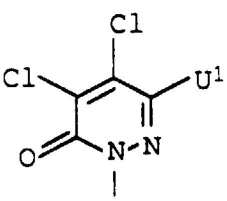 ,

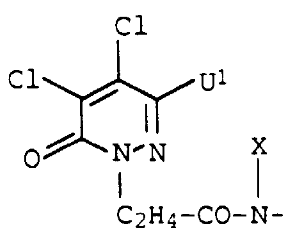

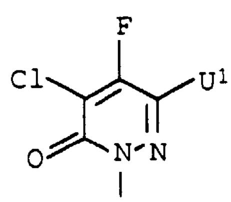 ,

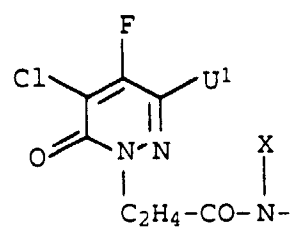 ,

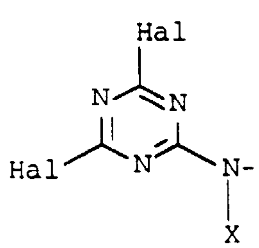

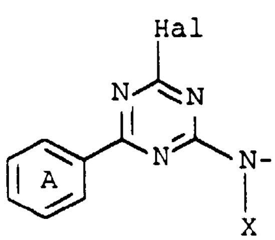 ,

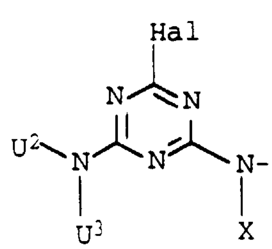 ,

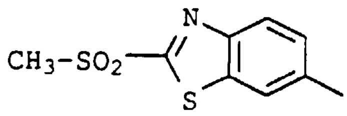 oder 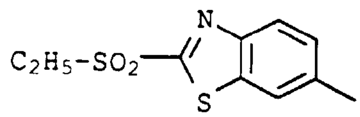 ,

worin

X Wasserstoff oder $C_1$-$C_4$-Alkyl,

Hal Fluor oder Chlor,

$U^1$ Wasserstoff oder Nitro und

$U^2$ und $U^3$ unabhängig voneinander jeweils Wasserstoff. $C_1$-$C_6$-Alkyl, das gegebenenfalls durch Hydroxy, Halogen, Cyano, Hydroxysulfonyl oder einen Rest der Formel -$SO_2$-Y, worin Y die obengenannte Bedeutung besitzt, substituiert ist und jeweils durch 1 oder 2 Sauerstoffatome in Etherfunktion, Imino- oder $C_1$-$C_4$-Alkyliminogruppen unterbrochen sein kann, oder $U^2$ und $U^3$ zusammen mit dem sie verbindenden Stickstoffatom, Pyrrolidinyl, Piperidinyl, Morpholinyl, Piperazinyl oder N-($C_1$-$C_4$-Alkyl)piperazinyl oder $U^2$ auch einen Rest der Formel

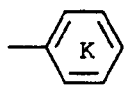

bedeuten,
wobei die Ringe A und K jeweils ein- oder zweifach durch Hydroxysulfonyl substituiert und benzoanelliert sein können und der Ring K davon unabhängig ein- oder zweifach durch Chlor, Nitro, $C_1$-$C_4$-Alkyl, $C_1$-$C_4$-Alkoxy, Cyano, Carboxyl, Acetylamino, Hydroxysulfonylmethyl oder einen Rest der Formel $CH_2$-$SO_2$-Y, $SO_2$-Y, NH-CO-Y oder $NU^2$-CO-$NU^2$-Z-$SO_2$-Y, worin Y und $U^2$ jeweils die obengenannte Bedeutung besitzen und Z für $C_2$-$C_6$-Alkylen, das gegebenenfalls durch Hydroxy, Chlor, Cyano, Carboxyl, $C_1$-$C_4$-Alkoxycarbonyl, $C_1$-$C_4$-Alkanoyloxy oder Sulfato substituiert ist und durch jeweils 1 oder 2 Sauerstoffatome in Etherfunktion oder Imino- oder $C_1$-$C_4$-Alkyliminogruppen unterbrochen sein kann, steht, substituiert sein kann.

Ankerreste $E^1$ und $E^2$ aus der aliphatischen Reihe sind beispielsweise Acryloyl-, Mono-, Di- oder Trichloracryloyl, Mono-, Di- oder Tribromacryloyl, -CO-CCl=CH-COOH, -CO-CH=CCl-COOH, 2-Chlorpropionyl, 1,2-Dichlorpropionyl, 1,2-Dibrompropionyl, 3-Phenylsulfonylpropionyl, 3-Methylsulfonylpropionyl, 2-Sulfatoethylaminosulfonyl, 2-Fluor-2-chlor-3,3-difluorcyclobut-1-ylcarbonyl, 2,2,3,3-Tetrafluorcyclobut-1-ylcarbonyl, 2,2,3,3-Tetrafluorcyclobut-1-ylsulfonyl, 2-(2,2,3,3-Tetrafluorcyclobut-1-yl)acryloyl, 1- oder 2-Alkyl- oder -Arylsulfonylacryloyl, wie 1- oder 2-Methylsulfonylacryloyl, oder ein Rest der Formel $SO_2$-Y, CONH-$W^1$-$SO_2$-Y oder NHCONH-$W^1$-$SO_2$-Y, worin $W^1$ und Y jeweils die obengenannte Bedeutung besitzen.

T in Formel I stellt ein Brückenglied dar. Geeignete Brückenglieder gehorchen z.B. der Formel

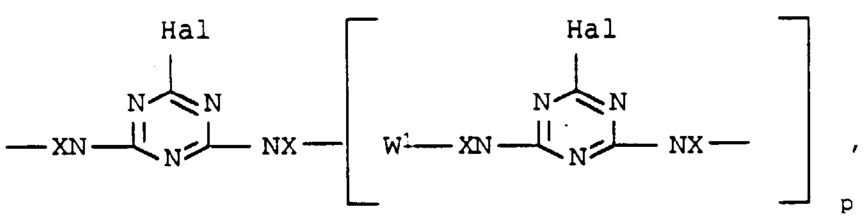 ,

—NX—Z—NX—, —NX—CO—NX—, CO oder $SO_2$,

worin p für 0 oder 1 steht und Hal, $W^1$, X und Z jeweils die obengenannte Bedeutung besitzen.

Besonders als Brückenglieder zu nennen sind Reste der Formel

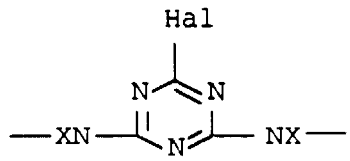

,

CO oder $SO_2$, wobei CO oder $SO_2$ hervorzuheben sind.

Wenn n 2 bedeutet, so gehorcht D vorzugsweise der Formel

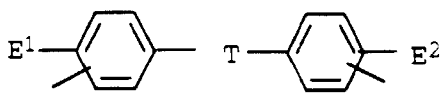

in der $E^1$, $E^2$ und T jeweils die obengenannte Bedeutung besitzen.

Bevorzugt sind Reaktivfarbstoffe der Formel I, in der $R^1$ Wasserstoff bedeutet.

Weiterhin bevorzugt sind Reaktivfarbstoffe der Formel I, in der $R^2$ Hydroxy bedeutet.

Weiterhin bevorzugt sind Reaktivfarbstoffe der Formel I, in der $R^3$ Hydroxysulfonylmethyl bedeutet.

Weiterhin bevorzugt sind Reaktivfarbstoffe der Formel I, in der $R^5$, $R^6$ und $R^7$jeweils Wasserstoff bedeuten.

Weiterhin bevorzugt sind Reaktivfarbstoffe der Formel I, in der $E^1$ für einen halogensubstituierten Rest von 1,3,5-Triazin oder einen Rest der Formel $SO_2$-Y, worin Y die obengenannte Bedeutung besitzt, steht.

Weiterhin bevorzugt sind Reaktivfarbstoffe der Formel I, in der, wenn n 2 ist, T für einen Rest der Formel CO oder $SO_2$ steht.

Weiterhin bevorzugt sind Reaktivfarbstoffe der Formel I, in der n 1 bedeutet.

Weiterhin bevorzugt sind Reaktivfarbstoffe der Formel I, in der $E^1$ für einen Rest der Formel

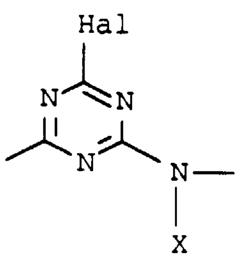

,

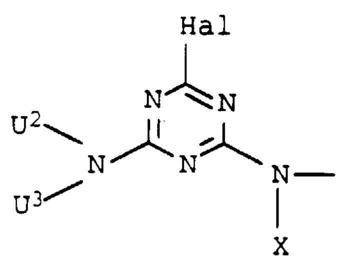

oder $SO_2$-Y

steht, worin Hal, $U^2$, $U^3$, X und Y jeweils die obengenannte Bedeutung besitzen.

Besonders bevorzugt sind Reaktivfarbstoffe der Formel I, in der $E^1$ für einen Rest der Formel $SO_2$-Y, worin Y die obengenannte Bedeutung besitzt, steht und sich in ortho-Position zur Azogruppe befindet.

Besonders bevorzugt sind weiterhin Reaktivfarbstoffe der Formel Ia

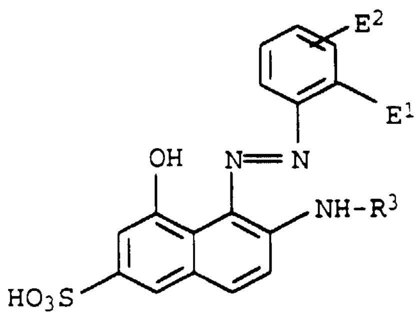

(Ia),

in der

$R^3$, $E^1$ und $E^2$ jeweils die obengenannte Bedeutung besitzen.

Von besonderem Interesse sind Reaktivfarbstoffe der Formel Ia, in der $E^1$ einen Rest der Formel $SO_2$-Y bedeutet.

Die neuen Reaktivfarbstoffe der Formel I können nach an sich bekannten Methoden erhalten werden.

Beispielsweise kann man ein Anilin der Formel IIa oder IIb

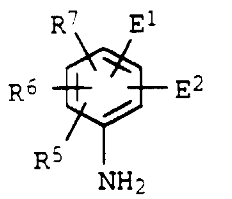

(IIa)

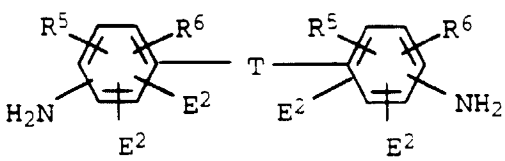

(IIb) ,

worin $R^5$, $R^6$, $R^7$, $E^1$, $E^2$ und T jeweils die obengenannte Bedeutung besitzen, auf an sich bekanntem Weg diazotieren oder tetrazotieren und mit einem Aminonaphthalin der Formel IIIa

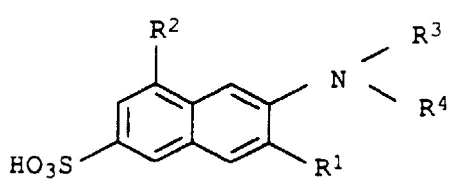

(IIIa),

in der $R^1$, $R^2$, $R^3$ und $R^4$ jeweils die obengenannte Bedeutung besitzen, kuppeln.

Ein weiterer Gegenstand der vorliegenden Anmeldung sind Aminonaphthaline der Formel III

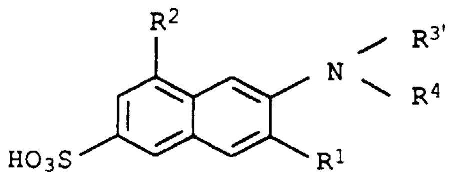

(III),

in der

$R^1$ Wasserstoff oder Hydroxysulfonyl,

$R^2$ Wasserstoff oder Hydroxy,

$R^{3'}$ Carboxymethyl, Hydroxysulfonylmethyl, einen Rest der Formel $W^1$-$SO_2$-Y oder $W^2$(-$SO_2$-Y)$_2$, worin $W^1$ für $C_1$-$C_4$-Alkylen oder gegebenenfalls substituiertes Phenylen, $W^2$ für einen 3-wertigen Rest eines Benzolringes, der gegebenenfalls substituiert ist, und Y für Vinyl oder einen Rest der Formel $C_2H_4$-Q stehen, wobei Q die Bedeutung einer unter alkalischen Reaktionsbedingungen abspaltbare Gruppe besitzt, Phenyl, das durch einen Rest der Formel CONH-$W^1$-$SO_2$-Y oder $SO_2$-Y, worin $W^1$ und Y jeweils die obengenannte Bedeutung besitzen, substituiert ist, und

$R^4$ Wasserstoff oder $R^4$ und $R^{3'}$ zusammen mit dem sie verbindenden Stickstoffatom einen 5- oder 6-gliedrigen gesättigten heterocyclischen Rest, der weitere Heteroatome aufweisen kann, bedeuten, mit der Massgabe, daß $R^{3'}$ nicht Carboxymethyl bedeutet, wenn R' für Wasserstoff und $R^2$ für Hydroxy stehen.

Bevorzugt sind Aminonaphthaline der Formel III, in der $R^1$ Wasserstoff bedeutet.

Weiterhin bevorzugt sind Aminonaphthaline der Formel III, in der $R^2$ Hydroxy bedeutet.

Weiterhin bevorzugt sind Aminonaphthaline der Formel III, in der $R^6$ Hydroxysulfonylmethyl bedeutet.

Die Aminonaphthaline der Formel III können nach an sich bekannten Methoden erhalten werden. Beispielsweise kann man ein Naphthalinderivat der Formel IV

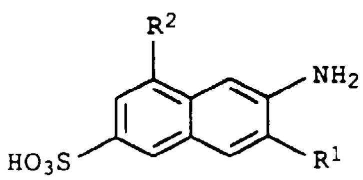

(IV),

in der $R^1$ und $R^2$ jeweils die obengenannte Bedeutung besitzen, in Gegenwart eines Alkalisulfits, z.B. Natrium- oder Kaliumsulfit, mit einem Amin der Formel V

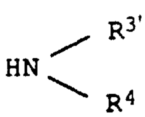

(V),

in der $R^4$ und $R^{3'}$ jeweils die obengenannte Bedeutung besitzen, zur Reaktion bringen.

Die neuen Reaktivfarbstoffe der Formel I eignen sich in vorteilhafter Weise zum Färben oder Bedrucken von Hydroxygruppen oder Stickstoffatome aufweisenden organischen Substraten. Solche Substrate sind beispielsweise Leder oder Fasermaterial, das überwiegend natürliche oder synthetische Polyamide oder natürliche oder regenerierte Cellulose enthält. Vorzugsweise eignen sich die neuen Farbstoffe zum Färben und Bedrucken von Textilmaterial auf der Basis von Wolle oder insbesondere von Baumwolle. Man erhält Ausfärbungen in roten Farbtönen.

Insbesondere auf Substraten auf Basis von Cellulose werden farbstarke Färbungen mit sehr hoher Fixierausbeute erhalten, die eine sehr gute Lichtechtheit sowie vorzügliche Naßechtheiten, wie Wasch-, Chlorbleich-, Peroxidbleich-, Alkali-, Meerwasser- oder Schweißechtheit, aufweisen.

Die folgenden Beispiele sollen die Erfindung näher erläutern.

Beispiel 1

0,1 mol der Verbindung der Formel

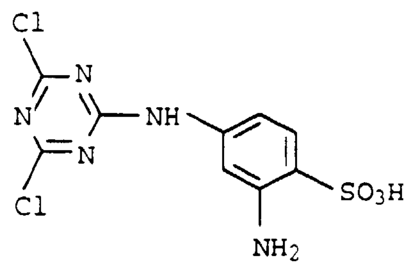

wurden in Form einer 35 gew.-%igen wäßrigen Lösung bei 0 bis 5°C mit 25 ml 30 gew.-%iger Salzsäure und 30 ml (0,1 mol) 23 gew.-%iger wäßriger Natriumnitritlösung diazotiert. Danach gab man 33,1 g (0,1 mol) der Verbindung der Formel

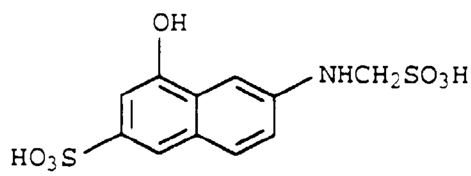

hinzu, wobei der pH-Wert durch Zugabe von wäßriger Natriumacetatlösung bei 2,5 bis 3 gehalten wurde. Nach beendeter Kupplung wurde mit Natriumhydrogencarbonat ein pH-Wert von 5 bis 5,5 eingestellt. Der Farbstoff der Formel

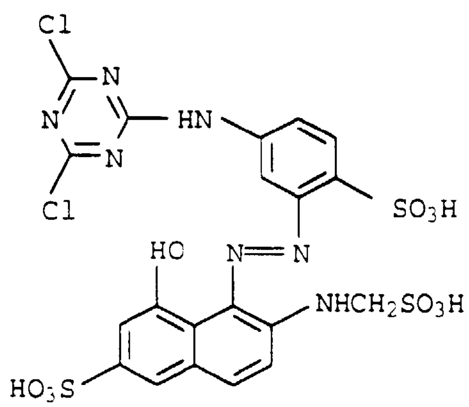

konnte durch Aussalzen, Abfiltrieren und Trocknen isoliert werden.
$\lambda_{max}$ (Wasser): 523 nm

Beispiel 2

28,1 g (0,1 mol) 1-Amino-2-(2-sulfatoethylsulfonyl)benzol wurden in 300 ml Eiswasser angerührt und durch Zugabe von 10 ml 30 gew.-%iger Salzsäure und 30 ml (0,1 mol) 23 gew.-%iger wäßriger Natriumnitritlösung diazotiert. Man hielt 1,5 h bei 0 bis 5°C. Danach gab man 34,3 g (0,103 mol) der Verbindung der Formel

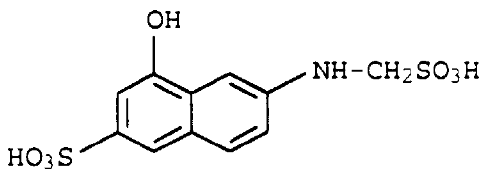

hinzu, wobei der pH-Wert durch Zugabe von wäßriger Natriumacetatlösung bei 2,5 gehalten wurde. Nach beendeter Kupplung wurde mit Natriumhydrogencarbonat ein pH-Wert von 5 bis 5,5 eingestellt. Der Farbstoff der Formel

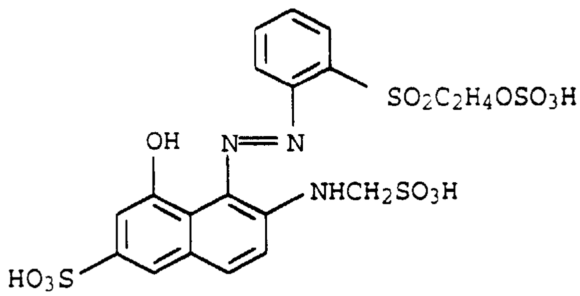

wurde durch Aussalzen, Abfiltrieren und Trocknen isoliert. Er kann ebenfalls durch Sprühtrocknung isoliert werden.
$\lambda_{max}$ (Wasser): 530 nm

Beispiel 3

0,1 mol der Verbindung der Formel

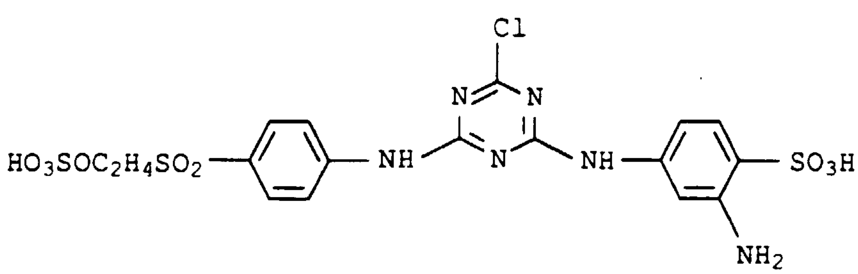

wurden in 200 ml Eiswasser angerührt. Durch Zugabe von 25 ml 30 gew.-%iger Salzsäure und 30 ml (0,1 mol) 23 gew.-%iger wäßriger Natriumnitritlösung diazotierte man bei 0 bis 5°C. Danach gab man 33,3 g (0,1 mol) der Verbindung der Formel

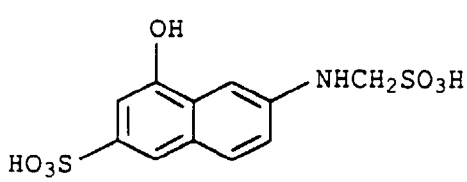

hinzu, wobei der pH-Wert durch Zugabe von wäßriger Natriumacetatlösung bei 2,5 bis 3 gehalten wurde. Nach beendeter Kupplung wurde mit Natriumhydrogencarbonat ein pH-Wert von 5 bis 5,5 eingestellt. Der Farbstoff der Formel

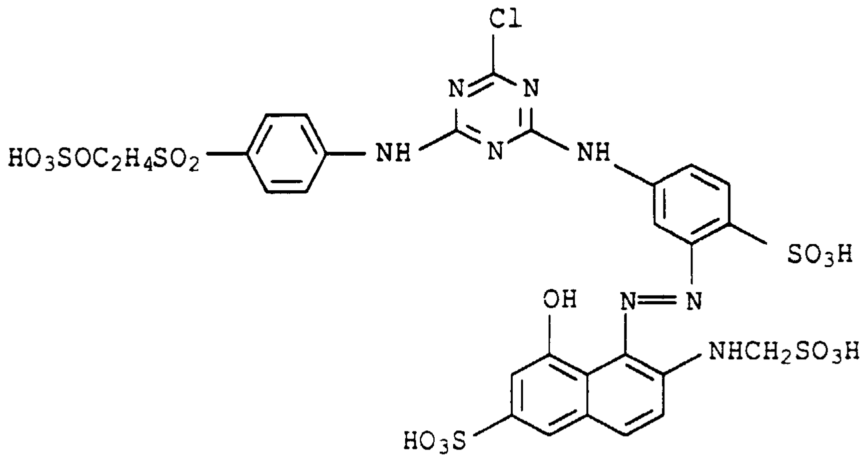

wurde durch Aussalzen, Abfiltrieren und Trocknen isoliert.
$\lambda_{max}$ (Wasser): 520 nm

Beispiel 4

Analog Beispiel 2 wurde der Farbstoff der Formel

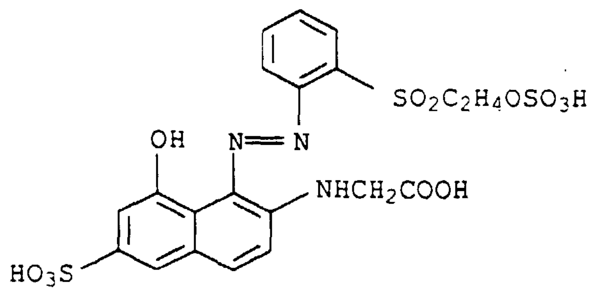

erhalten.
$\lambda_{max}$ (Wasser): 530 nm

Beispiel 5

27,0 g (0,042 mol) 3,3'-Diamino-4,4'-bis(2-sulfatoethylsulfonyl)benzophenon wurden in 200 ml Eiswasser angerührt, mit 30 ml 10 N Salzsäure und 100 ml Eisessig versetzt und unter Rühren bei einer Temperatur von 0 bis 5°C mit 30 ml 23 gew.-%iger wäßriger Natriumnitritlösung tetrazotiert. Nach 3 h Rühren bei 0 bis 5°C zersetzte man den geringen Überschuß an freier salpetriger Säure durch Zugabe von Amidosulfosäure. Dann wurden 17,4 ml (0,087 mol) einer wäßrigen Lösung der Verbindung der Formel

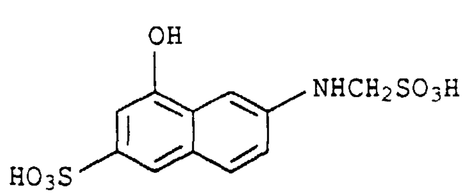

zugegeben und der pH-Wert mit Natriumacetat zwischen 3 bis 3,5 gehalten. Nach Beendigung der Reaktion ließ man auf Raumtemperatur erwärmen und stellte den pH-Wert mit Natriumcarbonat auf 5 bis 5,5. Die Reaktionslösung wurde mit Kaliumchlorid versetzt und 4 h gerührt. Der rote Niederschlag wurde abfiltriert und getrocknet. Man erhielt den

Farbstoff der Formel

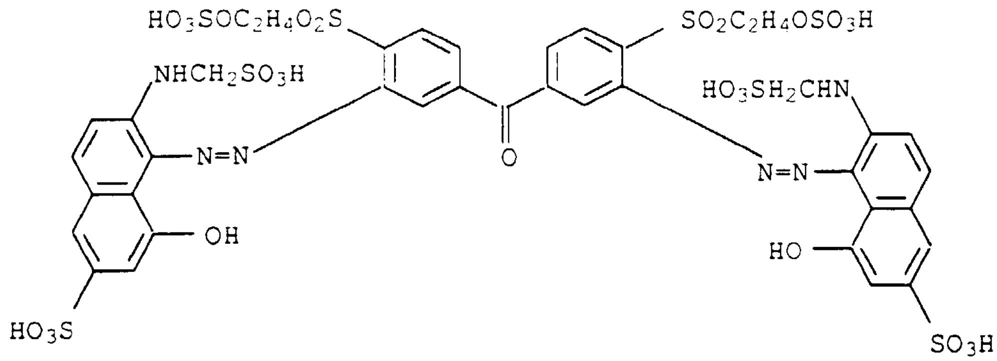

$\lambda_{max}$ (Wasser): 534 nm

In analoger Weise werden die im folgenden aufgeführten Farbstoffe erhalten.

Beispiel 6

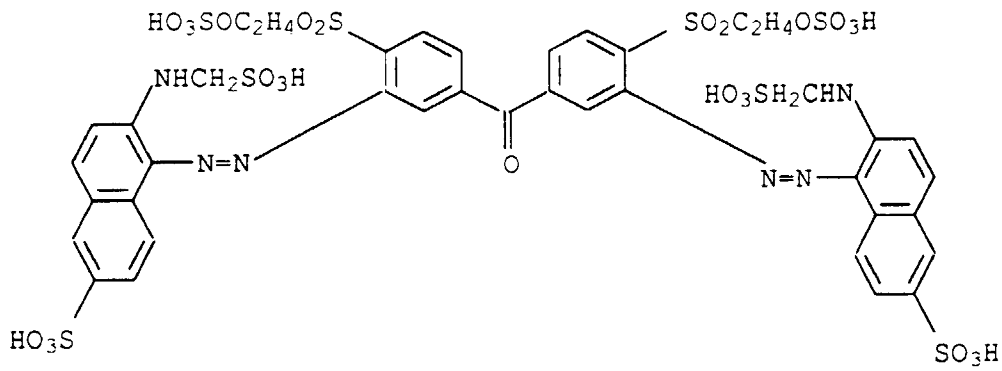

$\lambda_{max}$ (Wasser): 508 nm

Beispiel 7

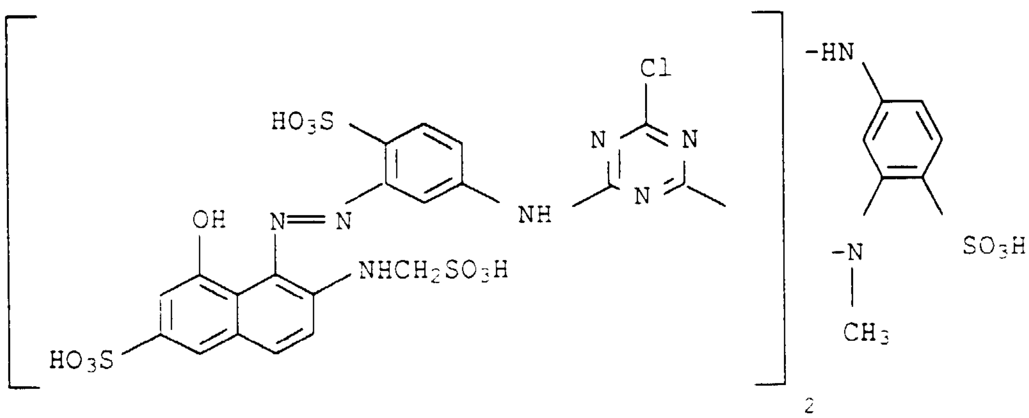

$\lambda_{max}$ (Wasser): 522 nm

Beispiel 8

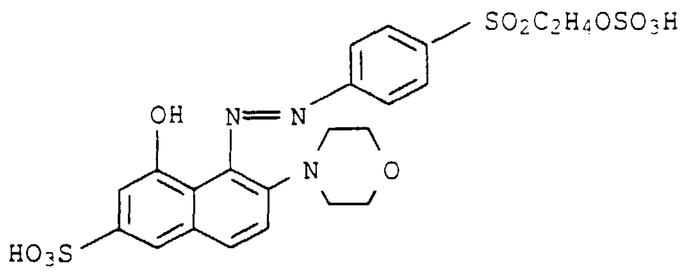

$\lambda_{max}$ (Wasser): 514 nm

Beispiel 9

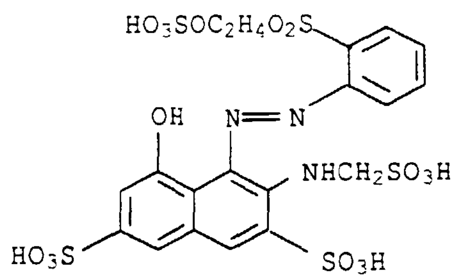

$\lambda_{max}$ (Wasser): 526 nm

Beispiel 10

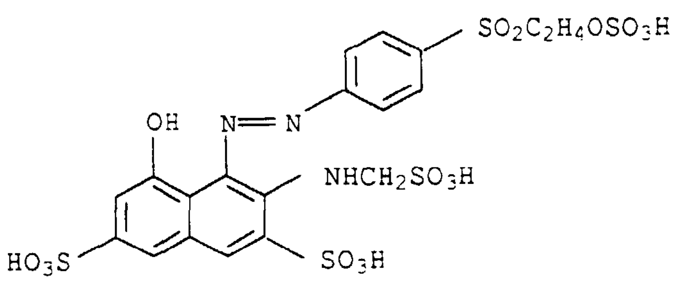

$\lambda_{max}$ (Wasser): 518 nm

Beispiel 11

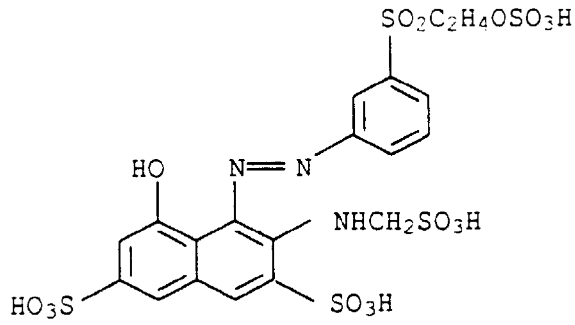

$\lambda_{max}$ (Wasser): 528 nm

Beispiele 12 und 13

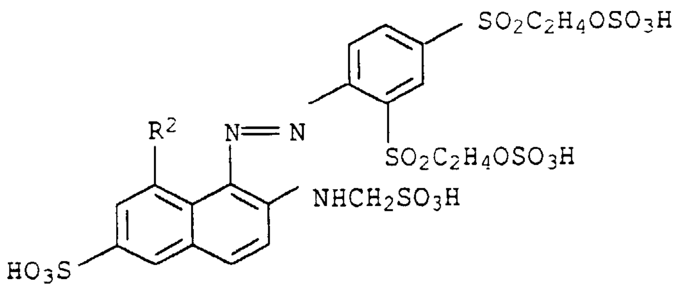

$R^2 = OH$ $\lambda_{max}$ (Wasser): 564 nm (Bsp. 12)
$R^2 = H$ $\lambda_{max}$ (Wasser): 532 nm (Bsp. 13)

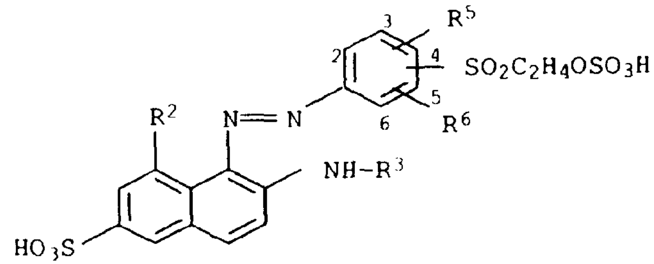

| Bsp. Nr. | $R^2$ | $R^1$ | Ringposition von $SO_2C_2H_4OSO_3H$ | $R^5$ und Ring-position | $R^6$ und Ring-position | λmax (in Wasser) [nm] |
|---|---|---|---|---|---|---|
| 14 | H | $CH_2SO_3H$ | 2 | H | H | 496 |
| 15 | H | $CH_2SO_3H$ | 3 | $SO_3H$ (6) | H | 522 |
| 16 | OH | 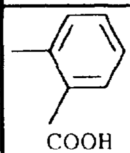 | 2 | H | H | 534 |
| 17 | OH | 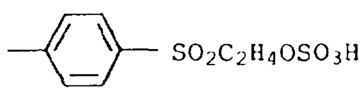 | 4 | H | H | 468 |
| 18 | OH | 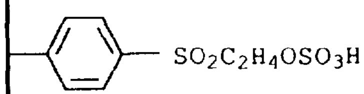 | 2 | H | H | 535 |

| Bsp. Nr. | $R^2$ | $R^3$ | Ringposition von $SO_2C_2H_4OSO_3H$ | $R^5$ und Ring-position | $R^6$ und Ring-position | λmax (in Wasser) [nm] |
|---|---|---|---|---|---|---|
| 19 | H | 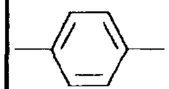 $SO_2C_2H_4OSO_3H$ | 4 | H | H | 506 |
| 20 | H | 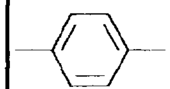 $SO_2C_2H_4OSO_3H$ | 2 | H | H | 508 |
| 21 | OH | $CH_2SO_3H$ | 3 | $SO_3H$ (4) | $SO_3H$ (6) | 494 |
| 22 | OH | $C_2H_4SO_2C_2H_4OSO_3H$ | 4 | H | H | 514 |
| 23 | OH | $C_2H_4SO_2C_2H_4OSO_3H$ | 3 | H | H | 506 |
| 24 | H | $CH_2SO_3H$ | 2 | $SO_3H$ (4) | H | 514 |
| 25 | OH | $CH_2SO_3H$ | 2 | $SO_3H$ (4) | H | 548 |
| 26 | H | $C_2H_4SO_2C_2H_4OSO_3H$ | 4 | H | H | 488 |
| 27 | H | 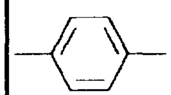 $SO_2C_2H_4OSO_3H$ | 3 | H | H | 494 |
| 28 | H | $C_2H_4SO_2C_2H_4OSO_3H$ | 2 | H | H | 493 |

| Bsp. Nr. | $R^2$ | $R^3$ | Ringposition von $SO_2C_2H_4OSO_3H$ | $R^5$ und Ring-position | $R^6$ und Ring-position | λmax (in Wasser) [nm] |
|---|---|---|---|---|---|---|
| 29 | H | $CH_2SO_3H$ | 3 | $SO_3H$ (4) | $SO_3H$ (6) | 514 |
| 30 | H | $CH_2SO_3H$ | 4 | $SO_3H$ (2) | $SO_3H$ (6) | 476 |
| 31 | OH | $CH_2SO_3H$ | 4 | $SO_3H$ (2) | $SO_3H$ (6) | 490 |

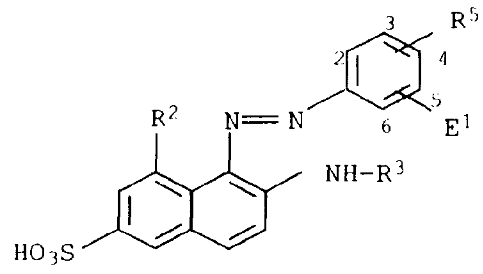

| Bsp. Nr. | $R^2$ | $R^3$ | $E^1$ und Ringposition | $R^5$ und Ring-position | λmax (in Wasser) [nm] |
|---|---|---|---|---|---|
| 32 | OH | $CH_2SO_3H$ | 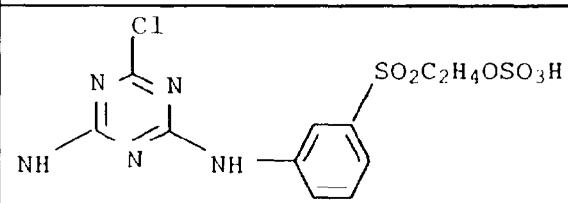 (3) | H | 518 |
| 33 | OH | $CH_2SO_3H$ | $NHCONHC_3H_6SO_2C_2H_4OSO_3H$ (3) | $SO_3H$ (6) | 520 |

| Bsp. Nr. | $R^2$ | $R^3$ | $E^1$ und Ringposition | $R^5$ und Ring-position | λmax (in Wasser) [nm] |
|---|---|---|---|---|---|
| 34 | OH | $CH_2SO_3H$ | 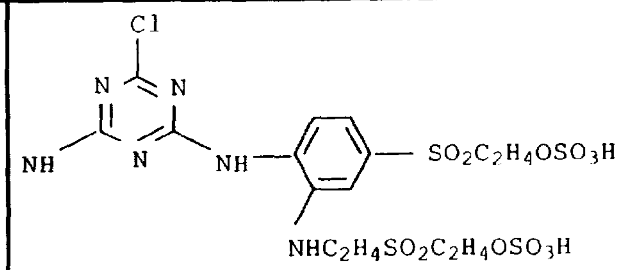 (3) | $SO_3H$ (2) | 522 |
| 35 | H | $CH_2SO_3H$ | 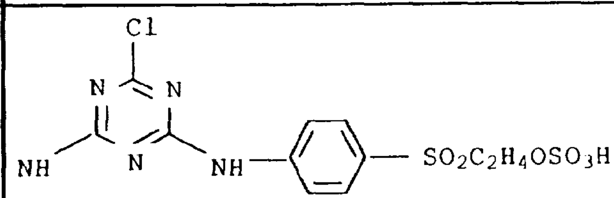 (3) | $SO_3H$ (6) | 484 |
| 36 | OH | $CH_2SO_3H$ | $CONHC_3H_6SO_2C_2H_4OSO_3H$ (4) | H | 520 |

## Patentansprüche

1. Reaktivfarbstoffe der Formel I

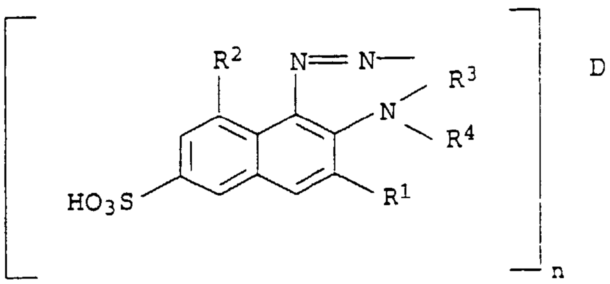 (I),

in der

n 1 oder 2,

$R^1$ Wasserstoff oder Hydroxysulfonyl,

$R^2$ Wasserstoff oder Hydroxy,

$R^3$ Carboxymethyl, Hydroxysulfonylmethyl, einen Rest der Formel $W^1$-$SO_2$-Y oder $W^2(-SO_2-Y)_2$, worin $W^1$ für $C_1$-$C_4$-Alkylen oder gegebenenfalls substituiertes Phenylen, $W^2$ für einen 3-wertigen Rest eines Benzolringes, der gegebenenfalls substituiert ist, und Y für Vinyl oder einen Rest der Formel $C_2H_4$-Q stehen, wobei Q die Bedeutung einer unter alkalischen Reaktionsbedingungen abspaltbaren Gruppe besitzt, Phenyl, das durch Carboxyl oder einen Rest der Formel CONH-$W^1$-$SO_2$-Y oder $SO_2$-Y, worin $W^1$ und Y jeweils die obengenannte Bedeutung besitzen, substituiert ist, oder wenn n 2 ist, auch Carboxy-$C_2$-$C_4$-alkyl oder Hydroxysulfonyl-$C_2$-$C_4$-alkyl,

$R^4$ Wasserstoff oder $R^3$ und $R^4$ zusammen mit dem sie verbindenden Stickstoffatom einen 5- oder 6-gliedrigen gesättigten heterocyclischen Rest, der weitere Heteroatome aufweisen kann, und

D , wenn n 1 ist, einen Rest der Formel

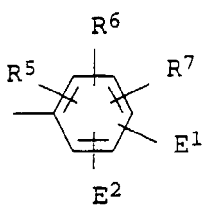

oder, wenn n 2 ist, einen Rest der Formel

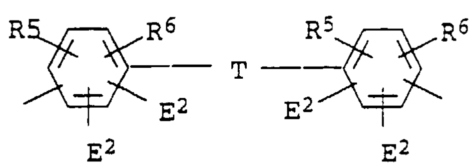 ,

worin $R^5$, $R^6$ und $R^7$ unabhängig voneinander jeweils für Wasserstoff, $C_1$-$C_4$-Alkyl, $C_1$-$C_4$-Alkoxy oder Hydroxysulfonyl, $E^1$ für einen heterocyclischen Ankerrest oder einen Ankerrest aus der aliphatischen Reihe, $E^2$ für Wasserstoff oder den Rest $E^1$ und T für ein Brückenglied stehen, bedeuten,

mit der Maßgabe, daß mindestens ein Ankerrest im Rest D vorhanden ist.

2. Reaktivfarbstoffe nach Anspruch 1, dadurch gekennzeichnet, daß $R^1$ Wasserstoff bedeutet.

3. Reaktivfarbstoffe nach Anspruch 1, dadurch gekennzeichnet, daß $R^2$ Hydroxy bedeutet.

4. Reaktivfarbstoffe nach Anspruch 1, dadurch gekennzeichnet, daß $R^3$ Hydroxysulfonylmethyl bedeutet.

5. Reaktivfarbstoffe nach Anspruch 1, dadurch gekennzeichnet, daß $R^5$, $R^6$ und $R^7$ jeweils Wasserstoff bedeuten.

6. Reaktivfarbstoffe nach Anspruch 1, dadurch gekennzeichnet, daß $E^1$ für einen halogensubstituierten Rest von 1,3,5-Triazin oder einen Rest der Formel $SO_2$-Y, worin Y die in Anspruch 1 genannte Bedeutung besitzt, steht.

7. Reaktivfarbstoffe nach Anspruch 1, dadurch gekennzeichnet, daß wenn n 2 ist, T für einen Rest der Formel CO oder $SO_2$ steht.

8. Reaktivfarbstoffe nach Anspruch 1, dadurch gekennzeichnet, daß n 1 bedeutet.

9. Reaktivfarbstoffe nach Anspruch 1, dadurch gekennzeichnet, daß $E^1$ für einen Rest der Formel $SO_2$-Y, worin Y die in Anspruch 1 genannte Bedeutung steht, bedeutet und sich in ortho-Position zur Azogruppe befindet.

10. Verwendung der Reaktivfarbstoffe gemäß Anspruch 1 zum Färben oder Bedrucken von Hydroxygruppen oder Stickstoffatome aufweisenden organischen Substraten.

11. Aminonaphthaline der Formel III

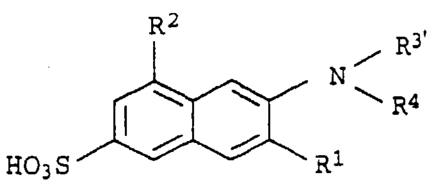

(III),

in der

$R^1$ Wasserstoff oder Hydroxysulfonyl,

$R^2$ Wasserstoff oder Hydroxy und

$R^{3'}$ Carboxymethyl, Hydroxysulfonylmethyl, einen Rest der Formel $W^1$-$SO_2$-Y oder $W^2(-SO_2-Y)_2$, worin $W^1$ für $C_1$-$C_4$-Alkylen oder gegebenenfalls substituiertes Phenylen, $W^2$ für einen 3-wertigen Rest eines Benzolringes, der gegebenenfalls substituiert ist, und Y für Vinyl oder einen Rest der Formel $C_2H_4$-Q stehen, wobei Q die Bedeutung einer unter alkalischen Reaktionsbedingungen abspaltbare Gruppe besitzt, Phenyl, das durch einen Rest der Formel CONH-$W^1$-$SO_2$-Y oder $SO_2$-Y, worin $W^1$ und Y jeweils die obengenannte Bedeutung besitzen, substituiert ist, und

$R^4$ Wasserstoff oder $R^4$ und $R^{3'}$ zusammen mit dem sie verbindenden Stickstoffatom einen 5- oder 6-gliedrigen gesättigten heterocyclischen Rest, der weitere Heteroatome aufweisen kann, bedeuten,

mit der Maßgabe, daß $R^{3'}$ nicht Carboxymethyl bedeutet, wenn $R^1$ für Wasserstoff und $R^2$ für Hydroxy stehen.

## Claims

1. A reactive dye of the formula I

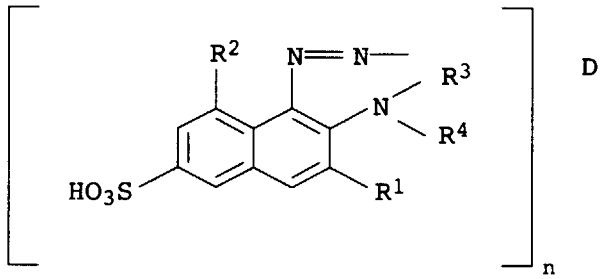 (I),

where

n is 1 or 2,

$R^1$ is hydrogen or hydroxysulfonyl,

$R^2$ is hydrogen or hydroxyl,

$R^3$ is carboxymethyl, hydroxysulfonylmethyl, a radical of the formula $W^1$-$SO_2$-Y or $W^2$(-$SO_2$-Y)$_2$, where $W^1$ is $C_1$-$C_4$-alkylene or unsubstituted or substituted phenylene, $W^2$ is a trivalent radical of a benzene ring which is unsubstituted or substituted and Y is vinyl or a radical of the formula $C_2H_4$-Q, where Q is a group which can be eliminated under alkaline reaction conditions, phenyl which is substituted by carboxyl or a radical of the formula CONH-$W^1$-$SO_2$-Y or $SO_2$-Y, where $W^1$ and Y each have the abovementioned meanings, or, if n is 2, $R^3$ may furthermore be carboxy-$C_2$-$C_4$-alkyl or hydroxysulfonyl-$C_2$-$C_4$-alkyl,

$R^4$ is hydrogen or $R^3$ and $R^4$, together with the nitrogen atom linking them, form a 5-membered or 6-membered saturated heterocyclic radical which may have further hetero atoms, and

D, if n is 1, is a radical of the formula

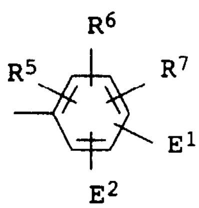

or, if n is 2, is a radical of the formula

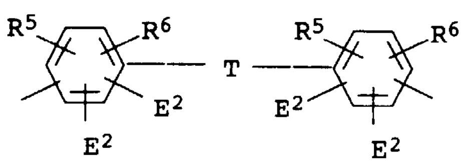 ,

where $R^5$, $R^6$ and $R^7$, independently of one another, are each hydrogen, $C_1$-$C_4$-alkyl, $C_1$-$C_4$-alkoxy or hydroxysulfonyl, $E^1$ is a heterocyclic mechanism or a mechanism from the aliphatic series, $E^2$ is hydrogen or $E^1$, and T is a bridge member,
with the proviso that at least one mechanism is present in D.

2. A reactive dye as claimed in claim 1, wherein $R^1$ is hydrogen.

3. A reactive dye as claimed in claim 1, wherein $R^2$ is hydroxyl.

**4.** A reactive dye as claimed in claim 1, wherein $R^3$ is hydroxysulfonylmethyl.

**5.** A reactive dye as claimed in claim 1, wherein $R^5$, $R^6$ and $R^7$ are each hydrogen.

**6.** A reactive dye as claimed in claim 1, wherein $E^1$ is a halogen-substituted radical of 1,3,5-triazine or a radical of the formula $SO_2$-Y, where Y has the meanings stated in claim 1.

**7.** A reactive dye as claimed in claim 1, wherein T is a radical of the formula CO or $SO_2$ if n is 2.

**8.** A reactive dye as claimed in claim 1, wherein n is 1.

**9.** A reactive dye as claimed in claim 1, wherein $E^1$ is a radical of the formula $SO_2$-Y, where Y has the meanings stated in claim 1, and is ortho to the azo group.

**10.** The use of a reactive dye as claimed in claim 1 for dyeing or printing hydroxyl-containing or nitrogen-containing organic substrates.

**11.** An aminonaphthalene of the formula III

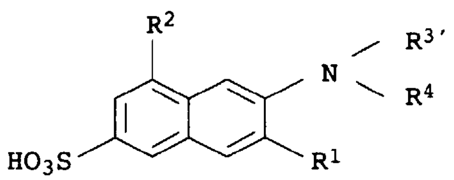

(III),

where

$R^1$ is hydrogen or hydroxysulfonyl,

$R^2$ is hydrogen or hydroxyl,

$R^{3'}$ is carboxymethyl, hydroxysulfonylmethyl or a radical of the formula $W^1$-$SO_2$-Y or $W^2$(-$SO_2$-Y)$_2$, where $W^1$ is $C_1$-$C_4$-alkylene or unsubstituted or substituted phenylene, $W^2$ is a trivalent radical of a benzene ring which is unsubstituted or substituted and Y is vinyl or a radical of the formula $C_2H_4$-Q, where Q is a group which can be eliminated under alkaline reaction conditions, phenyl which is substituted by a radical of the formula CONH-$W^1$-$SO_2$-Y or $SO_2$-Y, where $W^1$ and Y each have the abovementioned meanings, and

$R^4$ is hydrogen or $R^4$ and $R^{3'}$, together with the nitrogen atom linking them, form a 5-membered or 6-membered saturated heterocyclic radical which may have further hetero atoms, with the proviso that $R^{3'}$ is not carboxymethyl if $R^1$ is hydrogen and $R^2$ is hydroxyl.

**Revendications**

**1.** Colorants réactifs de formule I

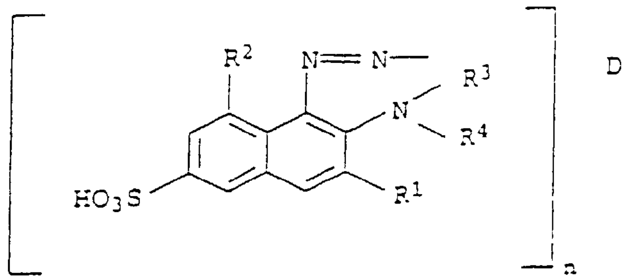

(I),

dans laquelle

n vaut 1 ou 2,

$R^1$ représente un atome d'hydrogène ou un groupement hydroxysulfonyle,

$R^2$ représente un atome d'hydrogène ou un groupement hydroxy,

$R^3$ représente un groupement carboxyméthyle, hydroxysulfonylméthyle, un reste de formule $W^1$-$SO_2$-Y ou $W^2$(-$SO_2$-Y$)_2$, où $W^1$ représente un groupement alkylène en $C_1$-$C_4$ ou un groupement phénylène éventuellement substitué, $W^2$ représente un reste trivalent d'un cycle benzènique, éventuellement substitué, et Y représente un groupement vinyle ou un reste de formule $C_2H_4$-Q, où Q prend la signification d'un groupement séparable dans des conditions réactionnelles alcalines, un groupement phényle substitué par un groupement carboxyle ou par un reste de formule CONH-$W^1$-$SO_2$-Y ou $SO_2$-Y, où $W^1$ et Y prennent chacun la signification susmentionnée, ou lorsque n vaut 2, un groupement carboxy(alkyle en $C_2$-$C_4$) ou hydroxysulfonyl(alkyle en $C_2$-$C_4$),

$R^4$ représente un atome d'hydrogène ou bien $R^3$ et $R^4$ représentent ensemble, avec l'atome d'azote qui les relie, un reste hétérocyclique saturé à 5 ou 6 maillons, pouvant contenir d'autres hétéroatomes, et

D représente, lorsque n vaut 1, un reste de formule

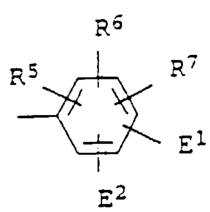

ou, lorsque n vaut 2, un reste de formule

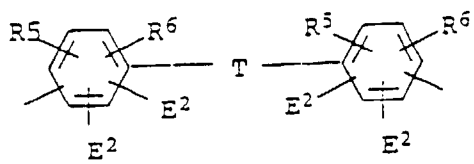

,

dans laquelle $R^5$, $R^6$ et $R^7$ représentent chacun indépendamment les uns des autres, un atome d'hydrogène, un groupement alkyle en $C_1$-$C_4$, un groupement alcoxy en $C_1$-$C_4$ ou hydroxysulfonyle, $E^1$ représente un reste d'ancrage hétérocyclique ou un reste d'ancrage issu de la série aliphatique, $E^2$ représente un atome d'hydrogène ou le reste $E^1$, et T est mis pour un maillon pontant,

étant spécifié qu'au moins un reste d'ancrage est présent dans D.

**2.** Colorants réactifs selon la revendication 1, caractérisés en ce que $R^1$ représente un atome d'hydrogène.

**3.** Colorants réactifs selon la revendication 1, caractérisés en ce que $R^2$ représente un groupement hydroxy.

**4.** Colorants réactifs selon la revendication 1, caractérisés en ce que $R^3$ représente un groupement hydroxysulfonylméthyle.

**5.** Colorants réactifs selon la revendication 1, caractérisés en ce que $R^5$, $R^6$ et $R^7$ représentent chacun un atome d'hydrogène.

**6.** Colorants réactifs selon la revendication 1, caractérisés en ce que $E^1$ représente un reste de 1,3,5-triazine substitué par des halogènes ou un reste de formule $SO_2$-Y, où Y prend la signification donnée dans la revendication 1.

**7.** Colorants réactifs selon la revendication 1, caractérisés en ce que lorsque n vaut 2, T représente un reste de formule CO ou $SO_2$.

**8.** Colorants réactifs selon la revendication 1, caractérisés en ce que n vaut 1.

**9.** Colorants réactifs selon la revendication 1, caractérisés en ce que $E^1$ représente un reste de formule $SO_2$-Y, où Y prend la signification mentionnée dans la revendication 1, et se trouve en position ortho par rapport au groupement azo.

**10.** Utilisation de colorants réactifs selon la revendication 1, pour la teinture ou l'impression de substrats organiques comportant des groupements hydroxy ou des atomes d'azote.

**11.** Aminonaphtalènes de formule III

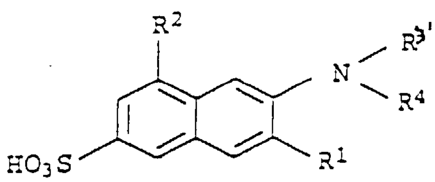 (III),

dans laquelle

$R^1$ représente un atome d'hydrogène ou un groupement hydroxysulfonyle,
$R^2$ représente un atome d'hydrogène ou un groupement hydroxy, et
$R^{3'}$ représente un groupement carboxyméthyle, hydroxysulfonylméthyle, un reste de formule $W^1$-$SO_2$-Y ou $W^2$(-$SO_2$-Y)$_2$, où $W^1$ représente un groupement alkylène en $C_1$-$C_4$ ou un groupement phénylène éventuellement substitué, $W^2$ représente un reste trivalent d'un cycle benzénique, éventuellement substitué, et Y représente un groupement vinyle ou un reste de formule $C_2H_4$-Q, où Q prend la signification d'un groupement séparable dans des conditions réactionnelles alcalines, un groupement phényle substitué par un reste de formule CONH-$W^1$-$SO_2$-Y ou $SO_2$-Y, où $W^1$ et Y prennent chacun la signification susmentionnée, et
$R^4$ représente un atome d'hydrogène ou bien $R^4$ et $R^{3'}$ représentent ensemble, avec l'atome d'azote qui les relie, un reste hétérocyclique saturé à 5 ou 6 maillons, pouvant contenir d'autres hétéroatomes,

étant spécifié que $R^{3'}$ ne représente pas un groupement carboxyméthyle lorsque $R^1$ représente un atome d'hydrogène et $R^2$ un groupement hydroxy.